# EUROPEAN PATENT APPLICATION

(11) **EP 4 686 457 A1**
(43) Date of publication of application: **04.02.2026**
(21) Application number: 24191560.2
(22) Date of filing: 29.07.2024
(51) Int. Cl.: A61F 2/00

(54) **CARTRIDGE-BASED SYSTEM TO STORE AND PROPEL STERILE IMPLANTS INTO THE BODY**

(71) Applicant: Clooxe, 1050 Bruxelles (BE)
(72) Inventor: DUISIT, Jérôme, 1050 Bruxelles (BE)
(74) Representative: Icosa Europe

(57) **Abstract**

The present invention relates to a sterile cartridge 100 comprising a rigid wall 110 defining a chamber, an inlet cover 140 closing an inlet open extremity 120, an outlet cover 150 closing an outlet open extremity 130, and an implant 10 disposed within the chamber.

## Description

### FIELD OF INVENTION

The present invention relates to a sterile device for packaging and inserting an implant.

More precisely, the present invention relates to a sterile cartridge comprising an implant and to an insertion device configured to nest the cartridge and to inject the implant in a subject. The present invention also relates to the manufacturing method of the sterile cartridge comprising the implant.

### BACKGROUND OF INVENTION

Various types of implants are used for either cosmetic or reconstructive surgery. Every known implant is supplied in a sealed, double sterile barrier primary package.

However, after the package comprising the implant has been opened to further perform the insertion of the implant in a subject, sterility of the implant may be broken. There is a direct correlation between implant manipulation risks, patient's skin contact, implant damage bacterial contamination, and capsular contracture.

Moreover, during the insertion, there is a risk of wrinkling or folding of the implant leading to mispositioning in the subject.

During insertion, forces should be applied on the implant implying a risk of implantation failure by gel fracture and/or implant rupture.

To minimize these drawbacks, implant inserters were developed to ensure a reduced patient's skin contact with the implant at the time of insertion, to reduce manual handling of the implant by the surgeon, to reach a better force distribution on the implant and to reduce the opening scar specifically in cosmetic surgery.

However, these known inserters are not satisfactory. Indeed, during the implant insertion procedure, a large number of manipulations are still needed before loading the implant in the inserter for the insertion. Indeed, the implant is provided independently from the inserter in a double sterile package which must be systematically opened. The implant is often covered by a disinfectant which should be rinsed before the insertion. The implant must also be examined for any particulate contamination, damage, or loss of shell integrity. The implant is often rinsed with a small amount of saline to remove the static. The inserter is also manipulated leading to a risk of contamination of the implant via the inserter. Indeed, the inserter is prefilled with a lubricating solution to facilitate the implant progression through the inserter. The implant is finally loaded by hands into the inserter. All these manipulations increase the risk of contamination.

Moreover, the inserters have a unique shape and are used for several implants. Therefore, the shape and dimensions of the inserters are not adapted to the shape and dimensions of the implants leading to an inhomogeneous application of the force during the insertion. There is thus a high risk of failure or wrong positioning of the implant.

There is thus a need for an implant which can be used with an implant inserter while reducing the risk of contamination, failure and/or wrong positioning.

In order to solve at last one of these problems, a purpose of this invention is to provide a sterile cartridge comprising the implant, the cartridge being loaded in an insertion device without manipulating the implant. The implant is inserted into the cartridge during the manufacturing procedure; the cartridge and the comprised implant being thus supplied in a sterile condition. The system of the invention thus takes the advantages of the insertion using an insertion device, *i.e.,* an easier, safer and faster implant insertion, while reducing or even eliminating the risk of contamination, failure and/or wrong positioning. Moreover, the sterile cartridge of the invention has a reduced volume compared to the double package leading to a reduction plastic used thanks to its shape which is adapted to the implant. This leads to a reduction of the transport and storage costs and to an eco-friendly cartridge. Finally, the use of a unique inserter for several cartridges also leads to a reduction of the cost and materials used for the insertion.

### SUMMARY

This invention thus relates to a sterile cartridge comprising:
- A rigid wall defining a chamber, the rigid wall comprising an inlet open extremity and an outlet open extremity, the rigid wall extending along a longitudinal axis passing through the inlet open extremity and the outlet open extremity,
- An inlet cover closing the inlet open extremity,
- An outlet cover closing the outlet open extremity, and
- An implant disposed within the chamber,
wherein at least part of the rigid wall fits at least part of the implant.

Indeed, the implant is already placed in a sterile cartridge which, thanks to its geometry, can be directly used for the insertion. The sterile cartridge thus takes the role of an implant packaging which can be directly used to insert the implant. Therefore, the implant does not have to be manipulated to take it out from a package and place it into an inserter. There is thus a reduction of the risk of contamination and failure. Moreover, thanks to the adaptation of the cartridge to the implant by the fitting of the rigid wall to the implant, there is no risk of displacement or wrong positioning of the implant.

The cartridge may be either used for large surgical incisions or mini-invasive surgical incisions. Moreover, the cartridge may be used for every type of implant.

Moreover, thanks to the fitting of the shape of the cartridge to the implant, the quantity of material used to manufacture the cartridge is reduced and optimized according to the implant. The volume and weight of the cartridge is thus reduced leading to a transport cost which is also reduced. This also leads to a reduction of material (such as plastic) consumption therefore leading to an economical and environment friendly device compared to known implants and their double packaging. Furthermore, since the cartridge is lighter, it is easier to use compared to the known inserters.

Finally, the use of the cartridge leads to a simplified insertion procedure. Indeed, only outlet cover and optionally the inlet cover have to be removed. The cartridge can be directly used either manually or using an inserter.

According to other advantageous aspect of the invention, a distance between the implant and the part of the rigid wall fitting at least part of the implant is ranging between 0 centimeter and 3 centimeters.

This distance is small enough to avoid any displacement of the implant and large enough to reduce the friction force allowing less force to be applied during the insertion. Moreover, the distance between the implant and the part of the rigid wall fitting at least part of the implant allows the injection of liquids or gel to cover the implant inside the cartridge.

According to other advantageous aspect of the invention, the rigid wall comprises a planar side extending from the inlet open extremity to the outlet open extremity.

The planar side is especially adapted for breast implants. Indeed, the planar side may be securely positioned on the chest of the subject during the insertion. Moreover, the planar side corresponds to the flat base of the dome-shaped breast implants. The cartridge is thus adapted to the shape of the implant.

According to other advantageous aspect of the invention, the cartridge further comprises a pushing block disposed within the chamber between the inlet open extremity and the implant, the pushing block being configured to slide along the longitudinal axis towards the outlet open extremity therefore letting the implant out through the outlet open extremity.

The pushing block allow to maintain the sterility inside the chamber. Indeed, the implant is pushed out from the cartridge by applying an external force on the pushing block avoiding any contact with the implant.

Moreover, it allows to distribute the force on a large surface of the implant thereby reducing the risk of failure.

According to other advantageous aspect of the invention, the cartridge comprises a built-in actuator fixed to the pushing block so that a force applied to the built-in actuator leads to a sliding of the pushing block.

Indeed, the built-in actuator allows a manual insertion of the implant inside a subject. This therefore leads to a fast insertion procedure because the cartridge is ready to use.

According to other advantageous aspect of the invention, at least part of an internal surface of the rigid wall is coated with a pro-sliding agent.

This pro-sliding (anti-adhesive or lubricating) lining of the internal surface allows to reduce the friction forces between the rigid wall and the implant. A smaller force is thus needed to insert the implant leading to a reduction of risk of failure.

According to other advantageous aspect of the invention, the implant is a deformable implant, the rigid external wall comprising an implant storage segment comprising the inlet open extremity, an inserter segment comprising the outlet open extremity and a compression segment linking the implant storage segment and the inserter segment, the implant storage segment fitting at least part of the deformable implant.

According to other advantageous aspect of the invention, the pushing block is disposed within the implant storage segment and is configured to be interlocked in the compression segment, the sliding of the pushing block along the longitudinal axis towards the outlet open extremity letting the deformable implant out through the outlet open extremity by deformation through the compression segment.

In other words, the pushing block has a specific shape corresponding to those of the inner surface of the compression segment. The pushing block and compression segment fit together at the end of the insertion of the implant so that the compression segment overlap the pushing block. This allows to expel nearly all the implant from the cartridge contrarily to a disk-shaped pushing block which is limited to a sliding inside the implant storage segment and is stopped at the compression segment.

According to other advantageous aspect of the invention, the implant is selected from the group of: a breast implant, a calf implant, gluteal implant, a sternal implant or a testicular implant.

This invention also relates to an insertion device configured to nest the sterile cartridge described hereabove, the insertion device comprising:
- a cartridge holder configured to nest the sterile cartridge and to lock the sterile cartridge in the cartridge holder, and
- an external actuator configured to apply a force on the implant in order to let the implant out through the outlet open extremity.

Indeed, the use of an insertion device leads to an easier, safer and faster implant insertion.

According to other advantageous aspect of the invention, the cartridge holder has an open configuration and a closed configuration, the open configuration allowing to load a sterile cartridge inside the cartridge holder or to remove a sterile cartridge from the cartridge holder.

This allows a safe enclosing of the cartridge which is locked in position during the use.

According to other advantageous aspect of the invention, the insertion device comprises a grasping element configured to transmit grasping force to the external actuator.

The grasping element allows an easier use of the insertion device compared to a piston. Moreover, the grasping element avoids the overpressure that can be applied on the implant when using a piston.

According to other advantageous aspect of the invention, the insertion device comprises a force reduction system.

This invention also relates to a kit of parts for performing implant insertion comprising the insertion device and the sterile cartridge described above, the sterile cartridge being disposed inside the cartridge holder.

According to other advantageous aspect of the invention, the sterile cartridge comprises a pushing block and the external actuator is configured to cooperate with the pushing block to transmit a force applied on the external actuator to the pushing block.

This invention also relates to a manufacturing method of a sterile cartridge, the method comprising:
- Constructing a rigid wall defining a chamber, the rigid wall comprising an inlet open extremity and an outlet open extremity; wherein at least part of the rigid wall is dimensioned in order to fit at least part of a natural shape of an implant;
- Placing said implant in the chamber,
- Closing the inlet open extremity with an inlet cover and closing the outlet open extremity with an outlet cover.

This allows to produce ready-to-use implants which remain sterile during the overall insertion process.

### DEFINITIONS

In the present invention, the following terms have the following meanings:

"**Fit**": the fitting between two elements of the device refers to the specific adaptation of the shape of one of the elements according to the shape of the other element. The shapes of the two elements are thus similar and overlap at least partially. The dimensions of one element are thus adapted to the dimensions of the other element while minimizing the space between the two elements.

"**Implant**" refers to an element designed to be inserted into a part of a subject's body to restore volume and/or shape of said body part. The implant can be definitive so that it is configured to stay in place in the body and to maintain its volume all along its lifetime or those of the subject. The implant can be temporary so that it is configured to be remove after a predefined period of time. The implant can be a testing implant (or sizer) configured to be inserted to the body part to evaluate the restored volume. The testing implant is removed before the insertion of the definitive or temporary implant. The implant can be progressive so that it is configured to progressively restore the volume of the body part by increasing its volume along the time. The implant can be deformable, semi-deformable or non-deformable (solid). The semi-deformable implant comprises a solid (non-deformable) part and a deformable part. The deformable implant or the deformable part of the semi-deformable implant may comprise, inside the elastomer silicone shell, a silicone gel, a saline solution or a structured and composite filler. The use of silicone gel allows to create asymmetric implants. The implant is thus deformable allowing to be inserted in the subject by a small surgical incision before recovering its undeformed shape thereby shaping the part of the subject into which it is inserted. This allows to reduce the size of the scar especially for cosmetic surgery. The deformable implant can be expandable, *i.e.,* inserted while being unfilled or little filled and then filled when in place in the body part. The expandable implant may comprise only the elastomer silicone shell which is filled from an exterior reservoir or by auto-filling with a silicone gel, a saline solution or a structured and composite filler once placed in the body. The deformable implant may also comprise a shape-memory material which recovers its definitive shape after insertion. The elastomer silicone shell may have an external surface which is smooth, semi-smooth, micro-textured, or macro-textured. The implant may be selected from the group of: a breast implant, a calf implant, a gluteal implant, a sternal implant, or a testicular implant. However, this list is non exhaustive and the sterile cartridge may be used with any kind of implant.

"**Natural shape of the implant**" refers to the shape of the implant before the insertion. It may correspond to the shape of the implant when it is not subjected to any external force. This corresponds to the maximal volume of the implant. Considering expandable implants (or expanders), the shape of the implant after the insertion inside a subject may be larger than the natural shape before insertion.

### DETAILED DESCRIPTION

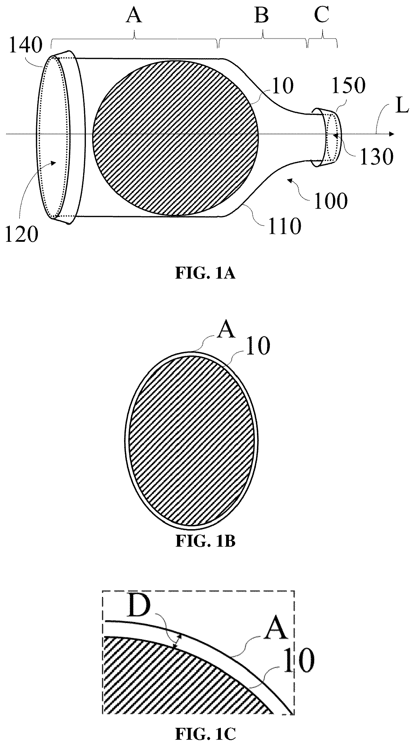

This invention relates to a sterile cartridge 100 comprising an implant 10. Examples of cartridges 100 are shown in figures 1A, 2A and 3A.

The cartridge 100 of the invention is advantageously adapted to the natural shape of the implant 10.

Indeed, the cartridge 100 comprises a rigid wall 110 defining a chamber.

The rigid wall 110 comprises an inlet open extremity 120 and an outlet open extremity 130.

The inlet open extremity 120 allows to place the implant 10 inside the chamber during the manufacture. The inlet open extremity 120 may be considered as the proximal extremity, *i.e.,* the extremity the farthest the implantation site. At the end of the manufacturing process and before the inserting process, the inlet open extremity 120 is closed by an inlet cover 140. The inlet cover 140 may be a rigid cover cooperating with the inlet open extremity 120 by clipping or screwing. The inlet cover 140 may alternatively be a flexible cover cooperating with the inlet open extremity 120 by gluing or sealing. This advantageously allows to pierce the inlet cover 140 with a syringe to inject additional liquid in the chamber before insertion.

The outlet open extremity 130 allows to expel the implant from the chamber and to insert it into the subject. The outlet open extremity 130 may be considered as the distal extremity, *i.e.,* the extremity the closest the implantation site. At the end of the manufacturing process and before the inserting process, the outlet open extremity 130 is closed by an outlet cover 150. The outlet cover 150 may be a rigid cover cooperating with the outlet open extremity 130 by clipping or screwing. The outlet cover 150 may alternatively be a flexible cover cooperating with the outlet open extremity 130 by gluing or sealing. This advantageously allow to pierce the outlet cover 150 with a syringe to inject additional liquid in the chamber before insertion. The outlet cover 150 is removed from the outlet open extremity 130 before the insertion of the implant 10.

The relative positions of the inlet open extremity 120 and the outlet open extremity 130 define a longitudinal axis L along which the rigid wall extends.

At least part of the rigid wall 110 fits at least part of the implant 10. In other words, the dimensions of the rigid wall 110 are adapted so that the implant 10 has its natural shape when placed in the chamber while minimizing the space between the implant 10 and the internal surface of the part of the rigid wall 110 fitting the implant 10. This avoids the movement of rotation or folding of the implant inside the chamber during the insertion or before the insertion (for example during the shipping of the cartridge).

To do so, the distance D between the implant 10 and the part of the rigid wall 110 fitting the implant 10, as represented for example in figure 1C, may range between 0 centimeter and 3 centimeters, preferably between 0.1 centimeters and 2 centimeters, even more preferably between 0.2 centimeters and 1 centimeter. This distance D corresponds to the minimum distance between the implant 10 and the rigid wall 110. Since the section of the implant 10 and possibly the section of the rigid wall 110 may vary along the longitudinal axis L, the distance D is computed based on the largest section of the implant 10 in its natural shape and the section of the rigid wall 110 at the longitudinal position of the largest section of the implant 10.

In other words, the volume of the part of the rigid wall 100 wherein the implant 10 is disposed is ranging between 100% and 200%, preferably between 100% and 120%, of the volume of the implant 10.

A non-zero distance between the implant 10 and the rigid wall 110 also allows the circulation of an injected solution, such as liquid or gel, around the implant before the insertion of the implant. For example, additional sterilizing agent or lubricant may be injected in order to coat the external surface of the implant 10.

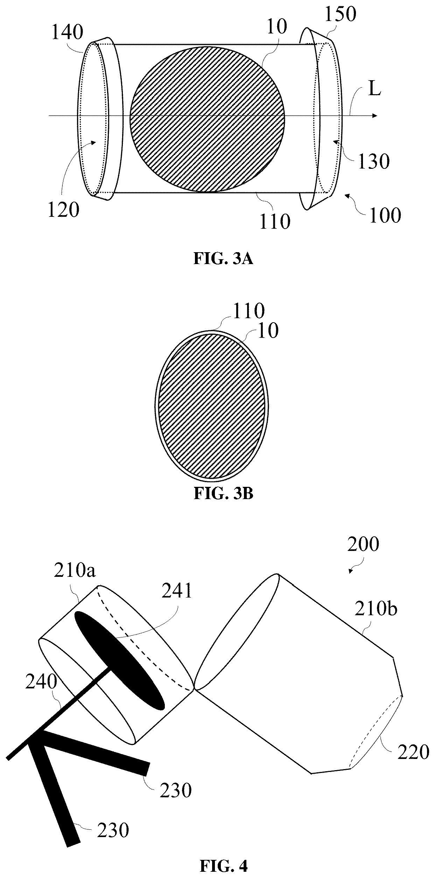

In the embodiment wherein the implant 10 is a solid implant, the rigid wall 110 preferably comprises a section which is constant along the longitudinal axis L. The rigid wall 110 thus forms a cylinder wherein the section fits the largest section of the implant 10 as represented in figures 3A and 3B. For example, for a spheroidal or ellipsoidal implant, the rigid wall 110 may present a circular or elliptical section as represented in figure 3B.

In the embodiment wherein the implant 10 is a deformable implant, the rigid wall 110 comprises an implant storage segment A comprising the inlet open extremity 120, an inserter segment C comprising the outlet open extremity 130 and a compression segment B linking the implant storage segment A and the inserter segment C. Examples of cartridges 100 comprising a deformable implant 10 are shown in figures 1A-2B.

In cosmetic surgery, it is advantageous that the outlet open extremity 130 has a small size in order to be introduced in a surgical incision which is as small as possible. The size of the outlet open extremity 130 may be of several centimeters to less than two centimeters in minimally invasive approaches. In reconstructive surgery, the size of the outlet open extremity 130 may reach the size of the natural shape of the implant and thus the size of the implant storage segment A.

The inlet open extremity 120 has a large size allowing to store the implant in it natural shape. For example, breast implants have a volume ranging from 105 to 1050 ml, a thickness ranging from 2.2 centimeters to 7.8 centimeters and a length measured perpendicularly to the thickness ranging from 8.5 centimeters to 15 centimeters. The inlet open extremity 120 may thus have a thickness ranging from 2.3 centimeters to 10 centimeters and a length ranging from 8.6 centimeters to 20 centimeters.

Therefore, the inlet open extremity 120 generally has a size larger than the outlet open extremity 130, especially for cosmetic surgery. Consequently, the compression segment B has a general shape of a funnel with a section reducing along the longitudinal axis L from the implant storage segment A to the inserter segment C.

The implant storage segment A fits at least part of the deformable implant 10.

The implant storage segment A may have a constant section along the longitudinal axis L. Alternatively, the implant storage segment A may have a shape which follows the shape of the distal portion of the natural shape of the deformable implant 10. In all embodiment for deformable implant, the implant storage segment A has a shape allowing the deformable implant 10 to be disposed in the implant storage segment A while being undeformed, *i.e.,* while being not submitted to any external force. On the contrary, in the compression segment B, the deformable implant 10 is deformed and compressed. The deformation and compression of the deformable implant 10 increases during the insertion by displacement along the longitudinal axis L towards the inserter segment C by the funnel-shaped compression segment B.

The deformable implant 10 may be disposed in the implant storage segment A during the manufacture without exerting any external force to the implant 10. In this embodiment, the deformable implant 10 has its natural shape in the implant storage segment A.

Alternatively, the deformable implant 10 may be preliminary compressed in the compression segment B during the manufacture. The insertion inside a subject thus corresponds to further displacement of the implant 10 along the longitudinal axis L towards the inserter segment C and thus further compression by the funnel-shaped compression segment B. In this embodiment of preliminary compression, the deformable implant 10 has its natural shape in the compression segment B. The natural shape of the deformable implant 10 is thus the shape of the compressed implant 10 before the insertion process in the subject, *i.e.,* before any force being applied by the practitioner to expel the implant 10. In this embodiment of preliminary compressed implant 10, the distance D between the implant 10 and the part of the rigid wall 110 fitting the implant 10 is ranging between 0 centimeter and 0.5°centimeters since the implant is compressed against the compression segment B. In this embodiment of preliminary compressed implant 10, the length of the implant storage segment A may be smaller than the length of the implant 10. The length of the implant storage segment A may even be of 0 centimeter leading to a rigid wall 110 comprising only the inserter segment C and the inlet open extremity 120 adjoining the compression segment B. In other words, in this embodiment, the compression segment B comprises the inlet open extremity 120.

For a spheroidal or ellipsoidal deformable implant 10, the implant storage segment A may present a circular or elliptical section as represented in figure 1B.

The inserter segment C may have a length along the longitudinal axis L ranging from 1 centimeter to 10 centimeters. An extension may advantageously be added on the inserter segment C to increase its length. The section of the outlet open extremity 130 may be circular, beveled or trumpet-shaped. The size of the outlet open extremity 130 preferably corresponds to the size of the surgical incision in the subject.

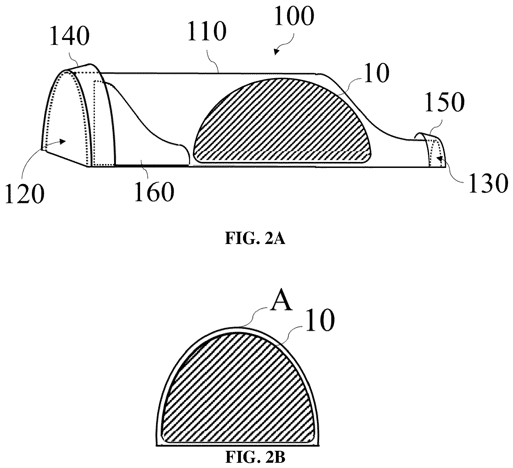

Breast implants - solid or deformable - are generally biconvex dome-shaped or semi-circular with a flat base. It has to be understood that the base of the implant is generally not strictly flat or planar but extends over a substantially planar area. The cartridges 100 into which these implants are comprised thus preferably present a planar side in their rigid wall 110 as represented in figure 2B. The planar side extends from the inlet open extremity 120 to the outlet open extremity 130 as represented in figure 2A.

At least part of an internal surface of the rigid wall 110 may be coated with a pro-sliding material, an anti-adhesive material and/or a lubricating material. This allows to reduce the force to be applied on the implant 10 during an insertion of the implant 10 in a subject.

The cartridge 100 may comprise a pushing block 160 disposed within the chamber between the inlet open extremity 120 and the implant 10. In the embodiment wherein the implant 10 is a deformable implant, the pushing block 160 is disposed within the implant storage segment A between the inlet open extremity 120 and the deformable implant 10. An example of cartridge 100 comprising a pushing block 160 is represented in figure 2A. The pushing block 160 is configured to slide along the longitudinal axis L towards the outlet open extremity 130 therefore letting the implant 10 out through the outlet open extremity 130 devoid of the outlet cover 150. In the embodiment wherein the implant 10 is a deformable implant, the deformable implant 10 is deformed through the compression segment B towards the outlet open extremity 130. The pushing block 160 may also slide towards the inlet open extremity 120.

The pushing block 160 may be completely rigid or may be at least partially deformable. For example, the pushing block 160 may comprise an elastomeric gasket, such as a rubber. The elastomeric gasket is fixed on the contour of the pushing block 160 to be in contact with the rigid wall 110. This is advantageous when the rigid wall 110 does not have a constant section along the longitudinal axis L.

The pushing block 160 may be in the form of a disc. Alternatively, the pushing block 160 may have an irregular shape on its distal side (*i.e.*, the side contacting the implant 10). For example, as represented in figure 2A, the shape of the pushing block 160 corresponds to the shape formed by the compression segment B. The pushing block 160 is thus configured to be interlocked in the compression segment B at the end of insertion. Thanks to this shape of the pushing block 160, the deformable implant 10 is pushed along the total or nearly total length of the cartridge 100. This thus avoids the risk of removal of the implant during the withdrawal of the cartridge 100 after the insertion.

### Manual configuration of the cartridge 100

The cartridge 100 may further comprise a built-in actuator fixed to the pushing block 160 so that a force applied to the built-in actuator leads to a sliding of the pushing block 160. The cartridge 100 is thus ready to use since, at the end of the manufacture, it comprises the implant 10 and the elements (built-in actuator and pushing block 160) allowing to manually expel the implant 10 after removing at least the outlet cover 150.

The built-in actuator is thus inserted at least partially in the chamber.

The built-in actuator may be a piston extending along the longitudinal axis L. In this piston embodiment, the built-in actuator may be inserted in the chamber and entirely covered with the inlet cover 140 before the use of the cartridge 100. The built-in actuator may be a pushing or sliding button disposed on the external surface of the rigid wall 110. In this pushing or sliding button embodiment, the built-in actuator may be inserted partially in the chamber and partially covered with the inlet cover 140 before the use of the cartridge 100.

The cartridge 100 may further comprise a force reduction system.

### Mechanical configuration of the cartridge 100

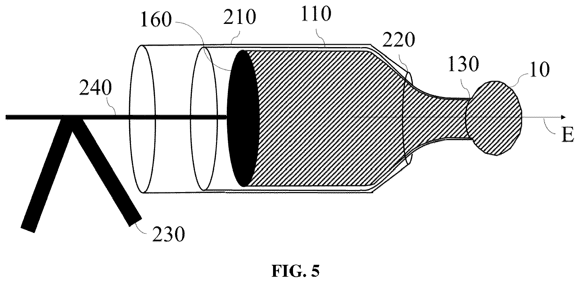

The invention also relates to an insertion device 200 used to mechanically expel the implant 10. Examples of insertion devices 200 are represented in figures 4 and 5.

The insertion device 200 comprises a cartridge holder 210 configured to nest the cartridge 100 and to lock the cartridge 100 in the cartridge holder 210.

The invention thus also relates to a kit of parts for performing implant insertion comprising the insertion device 200 and the cartridge 100, the cartridge 100 being disposed inside the cartridge holder 210.

The cartridge holder 210 may have a surface which, when the cartridge 100 is nested inside the insertion device 200, completely surrounds the rigid wall 110 except at the distal extremity wherein an opening 220 is disposed along the longitudinal axis L and is preferably aligned with the outlet open extremity 130. The opening 220 may be configured to let at least part of the rigid wall 110 comprising the outlet open extremity 130 (for example the inserter segment C) pass through as represented in figure 5. The opening 220 is thus disposed at the distal extremity of the insertion device 200. The outlet open extremity 130 thus protrudes outside the cartridge holder 210.

In the embodiment wherein the cartridge holder 210 completely surrounds the rigid wall 110, the cartridge holder 210 may be configured to be opened. Therefore, the cartridge holder 210 has an open configuration and a closed configuration. The open configuration allows to load the cartridge 100 inside the cartridge holder 210 or to remove the cartridge 100 from the cartridge holder 210. The cartridge 100 may be inserted or removed by a closable lateral opening disposed along the surface of the cartridge holder 210. The cartridge 100 may also be inserted or removed by the proximal extremity of the insertion device 200. Finally, the cartridge holder 210 may comprise a proximal part 210a and a distal part 210b. The proximal part 210a and the distal part 210b may be attached to each other in order to allow a rotation between the proximal part 210a and the distal part 210b as represented in figure 4. The proximal part 210a and the distal part 210b may be reversibly fixed: the proximal part 210a and the distal part 210b being uncoupled in the open configuration.

The cartridge holder 210 may have an open surface which, when the cartridge 100 is nested inside the insertion device 200, partially surrounds the rigid wall 110. The opening 220 overlaps at least partially the rigid wall 110 to lock the rigid wall 110 in the longitudinal axis L direction. For example, in the embodiment wherein the implant 10 is a deformable implant, the opening 220 may have a U-shape, a C-shape or a ring-shape configured to let the inserter segment C pass through. The cartridge holder 210 may have an open configuration and a closed configuration. The open configuration allows to load the cartridge 100 inside the cartridge holder 210 or to remove the cartridge 100 from the cartridge holder 210. The cartridge 100 may be inserted or removed by a closable lateral opening disposed along the surface of the cartridge holder 210 partially surrounding the rigid wall 110. The cartridge 100 may also be inserted or removed by the proximal extremity of the insertion device 200. Finally, the cartridge holder 210 may comprise a proximal part 210a and a distal part 210b. The proximal part 210a and the distal part 210b may be attached to each other in order to allow a rotation between the proximal part 210a and the distal part 210b. The proximal part 210a and the distal part 210b may be reversible fixed: the proximal part 210a and the distal part 210b being uncoupled in the open configuration.

The cartridge holder 210 may have a socket shape. The socket-shaped cartridge holder 210 may be configured to be screwed to the inlet open extremity 120.

The shape of the cartridge holder 210 may be configured to fit the shape of the rigid wall 110. Alternatively, the shape of the cartridge holder 210 may advantageously be a standard shape which is able to nest different sizes and shapes of cartridges 100. This allows to use only one single insertion device 200 for different implants.

The insertion device 200 comprises an external actuator 240 configured to apply a force on the implant 10 in order to let the implant 10 out through the outlet open extremity 130 devoid of the outlet cover 150. In the embodiment wherein the implant 10 is a deformable implant, the deformable implant 10 is let out by deformation through the compression segment B of the cartridge 100 thanks to the force applied by the external actuator 240 as illustrated in figure 5. Therefore, the external actuator 240 has dimensions allowing to be inserted inside the chamber.

The external actuator 240 may be a simple rod configured to slide parallelly to the longitudinal axis L of the cartridge 100 in order to exert a force on the implant 10. Preferably, the cartridge 100 comprises a pushing block 160. The external actuator 240 is therefore configured to cooperate with the pushing block 160, the force applied on the external actuator 240 leading to a slide of the pushing block 160 as represented in figure 5. This allows to distribute the force of the external actuator 240 on a large surface of the implant 10.

Alternatively or in combination, the external actuator 240 may comprise an external pushing block 241 which may be in contact with the implant 10 or with the pushing block 160.

The external pushing block 241 may be completely rigid or may be at least partially deformable. For example, the external pushing block 241 may comprise an elastomeric gasket, such as a rubber. The elastomeric gasket is fixed on the contour of the external pushing block 241 to be in contact with the rigid wall 110 of the cartridge 100. This is advantageous when the rigid wall 110 does not have a constant section along the longitudinal axis L.

The external pushing block 241 may be in the form of a disc as represented in figure 4. Alternatively, the external pushing block 241 may have an irregular shape on its distal side especially when no pushing block 160 is present in the cartridge 100. For example, in the embodiment wherein the implant 10 is a deformable implant, the shape of the external pushing block 241 corresponds to the shape formed by the compression segment B. The external pushing block 241 is configured to be interlocked in the compression segment B. Thanks to this shape, the deformable implant 10 may be pushed along the total length of the cartridge. This thus avoids the risk of removal of the implant during the withdrawal of the cartridge 100 after the insertion.

The insertion device 200 may further comprise a grasping element 230 configured to transmit grasping force to the external actuator 240. In the embodiment wherein the rigid wall 110 presents a planar side, the grasping element 230 may be positioned parallelly to the planar side. This allows the planar side to laid on the skin of the subject without being obstructed by the grasping element 230. This is particularly advantageous during the insertion of a breast implant through the infra-mammary fold and for reconstructions of the geometry of the chest well.

The insertion device 200 may further comprise a force reduction system.

The insertion device 200 may be used even if the cartridge 100 has a manual configuration. Indeed, the external actuator 240 may be configured to push on the built-in actuator fixed to the pushing block 160. This is advantageous because, in case of insertion device 200 failure, the cartridge 100 may still be used manually.

### Manufacturing method

Manipulating the implant 10 during the manufacture instead of during the procedure of insertion in a subject allows to ensure an industrial level of sterility which is higher than the sterility level of the surgical operation. Moreover, the implant is manipulated once, at the manufacture, and is never manipulated after.

The invention also relates to a manufacturing method of a sterile cartridge 100. For example, the implant 10 extends along an insertion axis E defined by the direction of the insertion of the implant 10 inside the subject.

The method comprises:
- Constructing a rigid wall 110, the rigid wall 110 comprising an inlet open extremity 120 and an outlet open extremity 130; wherein the at least part of the rigid wall 110 is dimensioned in order to fit at least part of a natural shape of an implant 10;
- Placing said implant 10 in the chamber, and
- Closing the inlet open extremity 120 with an inlet cover 140 and closing the outlet open extremity 130 with an outlet cover 150.

Preferably, the rigid wall 110 extends along a longitudinal axis L which, when the implant 10 is placed in the chamber, is parallel or overlaps the insertion axis E of the implant 10.

In the embodiment wherein the implant 10 is a deformable implant, the rigid external wall 100 may comprise an implant storage segment A comprising the inlet open extremity 120, an inserter segment C comprising the outlet open extremity 130 and a compression segment B linking the implant storage segment A and the inserter segment C. The implant storage segment A is dimensioned in order to fit at least part of a natural shape of the deformable implant 10. The deformable implant 10 is thus placed in the implant storage segment A.

The deformable implant 10 may be, during the placement in the implant storage segment, be preliminary compressed in the compression segment B. In this embodiment, the compression segment B may link the inlet open extremity 120 and the inserter segment C. The rigid wall 110 therefore does not comprise the implant storage segment A

In the embodiment wherein the cartridge 100 comprises a pushing block 160, the pushing block 160 is inserted in the chamber or the implant storage segment A after insertion of the implant 10 and before closing the inlet open extremity 120 with an inlet cover 140.

In the embodiment wherein the cartridge 100 comprises a built-in actuator, the built-in actuator is disposed at least partially in the chamber after insertion of the implant 10 and before closing the inlet open extremity 120 with an inlet cover 140.

The cartridge 100 manufactured by the method of the invention is sterile. For example, the rigid wall 110, the implant 10, the inlet cover 140 and outlet cover 150 and, if present, the pushing block 160 are individually sterilized before being assembled. Alternatively, the complete cartridge 100 is sterilized. The sterilization method may be a Dry Heat Sterilization method. The sterilization method may use radiation, for example, β-radiation, γ-radiation or UV light, with, preferably, a dose of at least 20 kGy. The sterilization method may use microwave-generated steam (MGS) or ultraviolet germicidal irradiation (UVGI). The sterilization method may use ortho-phthalaldehyde (OPA). The sterilization method may use, alone or in combination, heat, chemicals (hydrogen peroxide vapor, ethylene oxide, peroxides, ethylene oxide (EtO), liquid hydrogen peroxide (LHP), and hydrogen peroxide gas plasma (HPGP)), high pressure or filter.

Sterilizing agent or lubricant by be injected in the chamber before or after the closing the inlet open extremity 120 with an inlet cover 140 and the closing the outlet open extremity 130 with an outlet cover 150.

The rigid wall 110 and the pushing block 160 if present may comprise plastic or thermoplastic polyurethanes (TPU). The rigid wall 110 and the pushing block 160 if present may comprise polyethylene cross-linked foam and glycol-modified polyethylene terephthalate (PETG) because these compounds tend to be clear, easily sterilized, and cost effective. The rigid wall 110 and the pushing block 160 if present may comprise high density polyethylene such as TYVEK^{®}, a combination of TYVEK^{®} and polyethylene, SURLYN^{®}, a combination of SURLYN^{®} and TYVEK^{®}, metal foils, polymer films, polyvinyl chloride (PVC), polychloro-trifluoroethylene (PCTFE), cyclic olefin copolymers (COCs), and combinations thereof. These compounds advantageously resist to the sterilization.

The rigid wall 100 may comprise several superimposed layers. This leads to a rigid wall 100 which is abrasive resistant.

The rigid wall 110 may comprise some additive layers such as anti-blocking agents, inorganic or organic spacers, slip or separation aids, typically surface active, pigments or fillers and stabilizers.

The cartridge 100 may be packaged in an external packaging. Advantageously, the external packaging may have a shape corresponding to the cartridge 100 so that to reduce the quantity of material used.

### BRIEF DESCRIPTION OF THE DRAWINGS

**Figures 1A-1C** are a combination of figures. **Figure 1A** shows a representation of the cartridge 100 according to one embodiment of the invention wherein the deformable implant has an ellipsoidal shape. **Figure 1B** shows a sectional view of the cartridge 100 of Fig. 1A at the implant storage segment A. **Figure 1C** shows a zoom-in representation of the sectional view of Fig. 1B.
**Figures 2A-2B** are a combination of figures. **Figure 2A** shows a representation of the cartridge 100 according to one embodiment of the invention wherein the rigid wall comprises a planar side. **Figure 2B** shows a sectional view of the cartridge 100 of Fig. 2A at the implant storage segment A.
**Figures 3A-3B** are a combination of figures. **Figure 3A** shows a representation of the cartridge 100 according to one embodiment of the invention wherein the solid implant has an ellipsoidal shape. **Figure 3B** shows a sectional view of the cartridge 100 of Fig. 3A.
**Figure 4** shows a representation of the insertion device 200 according to one embodiment of the invention wherein the cartridge holder 210 comprises a proximal part 210a and the distal part 210b attached to each other in order to allow a rotation between the proximal part 210a and the distal part 210b. The configuration shown is open.
**Figure 5** shows a representation of the kit of part according to one embodiment of the invention in use. The deformable implant 10 is expelled in a direction E through the outlet open extremity 130.

### EXAMPLE

The present invention is further illustrated by the following example of use of the kit of part according to the invention.

The kit of parts of this example comprises a cartridge 100 comprising a biconvex semi-circular breast deformable implant of 350 milliliters comprising a flat base. The deformable implant 10 has an uncompressed shape presenting a diameter of 12 centimeters and a thickness of 3.8 centimeters.

The rigid wall 100 has a planar side.

The implant storage segment A have a shape which follows the shape of the distal portion of the uncompressed shape of the deformable implant 10. Therefore, the implant storage segment A defines a chamber with a maximum height of 5 centimeters and a maximum width measured on the planar side perpendicularly to the longitudinal axis L of 13 centimeters. The length of the implant storage segment A along the longitudinal axis L is 20 centimeters. The deformable implant has its natural shape in the implant storage segment A.

The outlet open extremity 130 has a semi-circular section of diameter of 2 centimeters.

The cartridge 100 has a pushing block 160 having a shape corresponding to the shape formed by the compression segment B.

The insertion device 200 has a cartridge holder 210 comprising a proximal part 210a and a distal part 210b. The external actuator 240 has an external pushing block 241 in the form of a disc. The insertion device 200 comprises a grasping element 230.

The insertion device 200 is firstly put in the open configuration of the cartridge holder 210 by rotating the proximal part 210a and a distal part 210b relatively to each other.

The inlet cover 140 and outlet cover 150 are removed from the inlet open extremity 120 and the outlet open extremity 130.

The cartridge 100 is then loaded in the distal part 210b of the cartridge holder 210. The outlet open extremity 130 is passing through the opening 220.

The insertion device 200 is then put in the closed configuration of the cartridge holder 210. The cartridge holder 210 completely surrounds the rigid wall 110 except at the opening 220.

An external force is applied on the grasping element 230 by a practitioner. The grasping element 230 transmits the grasping force to the external actuator 240. The external actuator 240 applies the force on the deformable implant 10 through the external pushing block 241 in contact with the pushing block 160.

The deformable implant 10 is deformed through the compression segment B and is expelled by the outlet open extremity 130 as shown in figure 5.

### NUMERICAL REFERENCES

10 -implant // 100 - Sterile cartridge // 110 - Rigid wall // 120 - Inlet open extremity // 130 - Outlet open extremity // 140 - Inlet cover // 150 - Outlet cover // 160 - Pushing block // 200 - Insertion device // 210 - Cartridge holder // 210a - Proximal part of the cartridge holder // 210b - Distal part of the cartridge holder // 220 - Opening // 230 - Grasping element // 240 - External actuator // 241 - External pushing block // A - implant storage segment // B - Compression segment // C - Inserter segment // D - Distance between rigid wall and the implant // E - Insertion axis // L - Longitudinal axis

## Claims

1. A sterile cartridge (100) comprising:
- A rigid wall (110) defining a chamber, the rigid wall (110) comprising an inlet open extremity (120) and an outlet open extremity (130), the rigid wall (110) extending along a longitudinal axis (L) passing through the inlet open extremity (120) and the outlet open extremity (130),
- An inlet cover (140) closing the inlet open extremity (120),
- An outlet cover (150) closing the outlet open extremity (130), and
- An implant (10) disposed within the chamber,
wherein at least part of the rigid wall (110) fits at least part of the implant (10).

2. The sterile cartridge (100) according to claim **1**, wherein a distance (D) between the implant (10) and the part of the rigid wall (110) fitting at least part of the implant (10) is ranging between 0 cm and 3 cm.

3. The sterile cartridge (100) according to claim **1** or **2**, wherein the rigid wall (110) comprises a planar side extending from the inlet open extremity (120) to the outlet open extremity (130).

4. The sterile cartridge (100) according to any one of claims **1** to **3**, further comprising a pushing block (160) disposed within the chamber between the inlet open extremity (120) and the implant (10), the pushing block (160) being configured to slide along the longitudinal axis (L) towards the outlet open extremity (130) therefore letting the implant (10) out through the outlet open extremity (130).

5. The sterile cartridge (100) according to claim **4**, further comprising a built-in actuator fixed to the pushing block (160) so that a force applied to the built-in actuator leads to a sliding of the pushing block (160).

6. The sterile cartridge (100) according to any one of claims **1** to **5**, wherein at least part of an internal surface of the rigid wall (110) is coated with a pro-sliding agent.

7. The sterile cartridge (100) according to any one of claims **1** to **6**, wherein the implant (10) is a deformable implant (10), the rigid external wall (110) comprising an implant storage segment (A) comprising the inlet open extremity (120), an inserter segment (C) comprising the outlet open extremity (130) and a compression segment (B) linking the implant storage segment (A) and the inserter segment (C), the implant storage segment (A) fitting at least part of the deformable implant (10).

8. The sterile cartridge (100) according to claim **7**, wherein the pushing block (160) is disposed within the implant storage segment (A) and is configured to be interlocked in the compression segment (B), the sliding of the pushing block (160) along the longitudinal axis (L) towards the outlet open extremity (130) letting the deformable implant (10) out through the outlet open extremity (130) by deformation through the compression segment (B).

9. The sterile cartridge (100) according to any one of claims **1** to **8**, wherein the implant (10) is selected from the group of: a breast implant, a calf implant, a gluteal implant, a sternal implant, or a testicular implant.

10. An insertion device (200) configured to nest the sterile cartridge (100) according to any one of claims **1** to **9**, the insertion device comprising:
- a cartridge holder (210) configured to nest the sterile cartridge (100) and to lock the sterile cartridge (100) in the cartridge holder (210), and
- an external actuator (240) configured to apply a force on the implant (10) in order to let the implant (10) out through the outlet open extremity (130).

11. The insertion device (200) according to claim **10**, wherein the cartridge holder (210) has an open configuration and a closed configuration, the open configuration allowing to load a sterile cartridge (100) inside the cartridge holder (210) or to remove a sterile cartridge (100) from the cartridge holder (210).

12. The insertion device (200) according to any one of claims **10** to **11**, further comprising a grasping element (230) configured to transmit grasping force to the external actuator (240).

13. A kit of parts for performing implant insertion comprising the insertion device (200) according to anyone of claims **10** to **12** and the sterile cartridge (100) according to anyone of claims **1** to **9**, the sterile cartridge (100) being disposed inside the cartridge holder (210).

14. The kit of parts according to claim **13**, wherein the sterile cartridge (100) comprises a pushing block (160) and the external actuator (240) is configured to cooperate with the pushing block (160) to transmit a force applied on the external actuator (240) to the pushing block (160).

15. A manufacturing method of a sterile cartridge (100), the method comprising:
- Constructing a rigid wall (110) defining a chamber, the rigid wall (110) comprising an inlet open extremity (120) and an outlet open extremity (130); wherein at least part of the rigid wall (110) is dimensioned in order to fit at least part of a natural shape of an implant (10);
- Placing said implant (10) in the chamber, and
- Closing the inlet open extremity (120) with an inlet cover (140) and closing the outlet open extremity (130) with an outlet cover (150).
